# FASCICULE DE BREVET EUROPEEN

(11) **EP 0 443 892 B1**
(45) Date de publication et mention de la délivrance du brevet: **26.04.1995**
(21) Numéro de dépôt: 91400203.5
(22) Date de dépôt: 29.01.1991
(51) Int. Cl.: A61B 17/58

(54) **Implant pour dispositif d'ostéosynthèse en particulier du rachis**
Implantat für Osteosyntheseeinrichtung, insbesondere für die Wirbelsäule
Implant for osteosynthesis device, in particular for the spinal column

(30) Priorité: 19.02.1990 FR 9001972
(43) Date de publication de la demande: 28.08.1991
(73) Titulaire: SOCIETE DE FABRICATION DE MATERIEL ORTHOPEDIQUE SOFAMOR, 75016 Paris (FR)
(72) Inventeur: Cotrel, Yves, F-75015 Paris (FR)
(74) Mandataire: Martin, Jean-Paul

(56) Documents cités:
- EP-A- 0 348 272
- FR-A- 2 559 378
- FR-A- 2 624 720

## Description

La présente invention a pour objet un implant pour dispositif d'ostéosynthèse, en particulier du rachis, du type comprenant une partie destinée à l'ancrage osseux et un corps de fixation sur une tige, dans lequel le corps présente un canal débouchant sur une partie postérieure du corps, délimitant deux branches latérales et ouvert de part et d'autre du corps pour pouvoir recevoir la tige; cet implant comprend également un bouchon fileté adapté pour pouvoir être vissé dans un taraudage formé dans les parois intérieures des deux branches latérales afin de bloquer la tige en translation et en rotation.

Un tel implant est décrit dans la demande de brevet français 88 08 538 (FR-A-2 633 177) déposée le 24 juin 1988 par la Demanderesse. Ce dispositif, qui permet la fixation d'une tige moletée à une vertèbre, présente certains inconvénients :
- d'une part on constate une certaine difficulté pour déplacer la tige après mise en place du bouchon fileté de fixation.
- d'autre part les branches du corps en U peuvent subir un certain écartement lors du serrage du bouchon sur la tige.

L'invention a donc pour but de remédier à ces inconvénients.

Suivant l'invention, l'implant comprend une plaquette de liaison entre le bouchon et la tige, munie de moyens de fixation aux branches du corps.

Ainsi lors du serrage du bouchon, la plaquette s'applique sur la tige et l'immobilise complètement dans le corps de l'implant, en même temps que les moyens de fixation de la plaquette aux branches du corps s'opposent à tout écartement desdites branches.

Suivant un mode de réalisation de l'invention, les moyens de fixation de la plaquette aux branches sont formés par une bague entourant lesdites branches et solidaire des extrémités de la plaquette, qui s'étend d'une extrémité à l'autre du canal du corps, les branches du corps pouvant être introduites dans des passages réservés entre la bague et les bords de la plaquette.

La plaquette et la bague peuvent être venues de matière, la bague enserrant les deux branches et s'opposant à leur écartement lors du vissage du bouchon fileté.

L'invention sera maintenant décrite en référence aux dessins annexés qui en illustrent une forme de réalisation à titre d'exemple non limitatif.

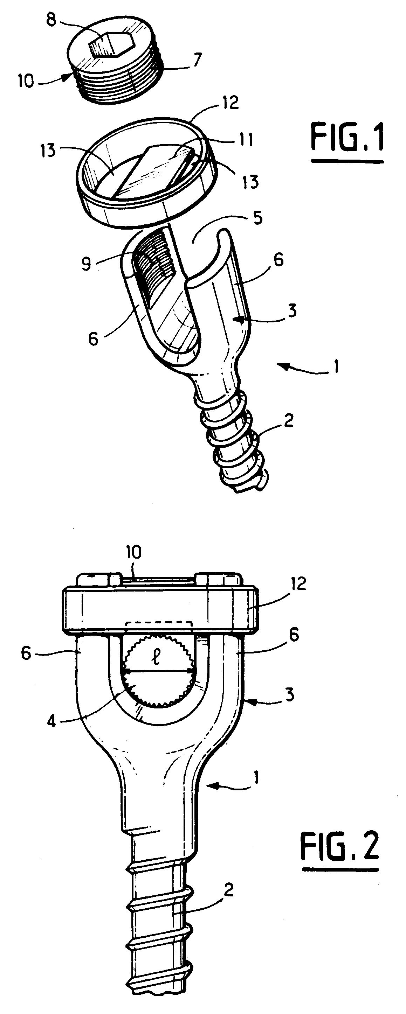

La figure 1 est une vue en perspective éclatée d'une forme de réalisation de l'implant selon l'invention.

La figure 2 est une vue en élévation de l'implant de la Fig. 1 équipé de la tige correspondante bloquée dans le canal du corps par la pastille et le bouchon.

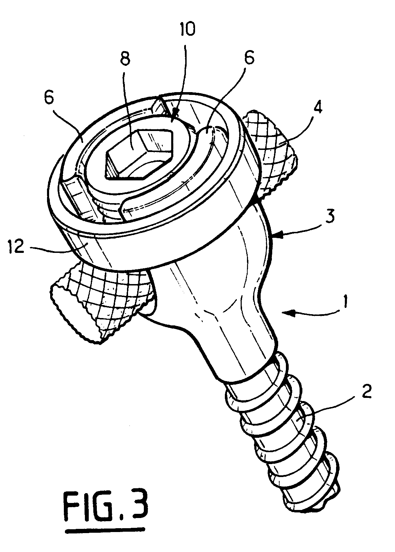

La figure 3 est une vue en perspective de l'implant de la Fig. 2 et d'un tronçon de la tige correspondante.

L'implant 1 représenté aux dessins est destiné à un dispositif d'ostéosynthèse non représenté, en particulier du rachis. Il comprend une partie 2, destinée à l'ancrage osseux, ici constituée par une tige fileté, qui peut être remplacée par un crochet comme dans l'implant décrit dans précitée FR-A-2 633 177.

L'implant comprend également un corps 3 de fixation sur une tige 4 à aspérités, par exemple une tige moletée ou à pointes de diamant. Le corps 3 présente un canal 5 débouchant sur une partie postérieure du corps, délimitant deux branches latérales 6 et ouvert de part et d'autre du corps pour pouvoir recevoir la tige 4. Le corps 3 a ainsi une section en U dans un plan transversal au canal 5.

L'implant est également équipé d'un bouchon 10 pourvu d'un filetage 7 et dans lequel est usiné un évidement 8 convenablement profilé pour recevoir un outil correspondant de vissage du bouchon 10 dans des taraudages 9 des parois intérieures des branches 6. L'implant 1 comprend une plaquette 11 de liaison entre le bouchon 6 et la tige 4, s'étendant d'une extrémité à l'autre du canal 5 et dont la largeur est légèrement inférieure à la largeur 1 de l'entrée du canal 5. Les extrémités de la plaquette 11 sont solidaires d'une bague 12, de préférence cylindrique, qui entoure les deux branches 6 lorsqu'elle est mise en place sur le corps 3, par insertion des branches 6 dans les passages 13 réserves entre la paroi intérieure de la bague 12 et les bords longitudinaux de la plaquette 11.

La plaquette 11 et la bague 12 peuvent être rapportées l'une à l'autre, ou de préférence venues de matière en formant une pièce monobloc. La plaquette 11 relie deux portions diamétralement opposées du bord de la bague cylindrique 12 tourné vers les fond du canal 5.

La mise en place des divers éléments constitutifs de cet implant et le blocage de la tige 4 s'effectuent de manière très simple : on introduit d'abord la tige 4 dans le canal 5, puis on enfile la bague 12 autour des extrémités des branches 6 jusqu'à ce que la plaquette ou pastille 11 vienne en appui sur la tige 4, les branches 6 s'intercalant entre la bague 12 et les bords longitudinaux de la plaquette 11. Enfin on introduit le bouchon 10 entre les extrémités des branches 6, et on le visse dans les taraudages 9 jusqu'à ce que sa face plane opposée à la cavité 8 vienne se bloquer sur la plaquette 11. Cette dernière est ainsi appliquée fermement sur la surface à aspérités de la tige 4, laquelle est bloquée en translation et en rotation.

Il n'est plus nécessaire avec un tel dispositif de ménager des moyens d'accrochage sur la face du bouchon 10 orientée vers la tige 4, contrairement à ce qui est prévu dans le brevet français 2 633 177. Par ailleurs il est possible de déplacer légèrement la tige pour lui faire trouver sa position définitive après mise en place de la plaquette 11 et de la bague 12, et avant serrage du bouchon 10. Enfin la hauteur de la bague 12 peut varier.

## Revendications

1. Implant (1) pour dispositif d'ostéosynthèse, en particulier du rachis, comprenant une partie (2) destinée à l'ancrage osseux et un corps (3) de fixation sur une tige (4), dans lequel le corps présente un canal (5) débouchant sur une partie postérieure du corps, délimitant deux branches latérales (6) et ouvert de part et d'autre du corps pour pouvoir recevoir la tige, cet implant comprenant également un bouchon fileté (10) adapté pour pouvoir être vissé dans des taraudages (9) formés sur les parois intérieures des deux branches latérales (6) afin de bloquer la tige (4) en translation et en rotation, caractérisé en ce qu'il comprend une plaquette (11) de liaison entre le bouchon (10) et la tige (4), munie de moyens (12) de fixation aux branches du corps.

2. Implant selon la revendication 1, caractérisé en ce que les moyens de fixation de la plaquette (11) aux branches (6) sont formés par une bague (12) entourant lesdites branches et solidaire des extrémités de la plaquette, qui s'étend d'une extrémité à l'autre du canal (5) du corps (3), les branches du corps pouvant être introduites dans des passages (13) réservés entre la bague (12) et les bords de la plaquette (11).

3. Implant selon la revendication 2, caractérisé en ce que la face du bouchon (10) en appui sur la plaquette (11) est plane.

## Claims

1. Implant (1) for an osteosynthesis device, particularly for the spine, comprising a post (2) intended for anchoring in the bone and a member (3) for attachment to a rod (4), in which the member has a channel (5) opening onto a rear part of the member, defining two lateral branches (6) and open on both sides of said member so as to be able to receive the rod, this implant also having a threaded plug (10) adapted to be screwed into screw threads (9) formed on the inner walls of the two lateral branches (6) in order to secure the rod (4) against translational and rotational movement, characterized in that it comprises a small connecting plate (11) between the plug (10) and the rod (4), provided with means (12) for attachment to the branches of the member.

2. Implant according to claim 1, characterized in that the means for attaching the plate (11) to the branches (6) consist of a ring (12) encircling said branches and integrally fixed to the ends of the plate, which extends from one end of the channel (5) in the member (3) to the other, the branches of the member being insertable into passages (13) kept free between the ring (12) and the edges of the plate (11).

3. Implant according to claim 2, characterized in that the surface of the plug (10) abutting on the plate (11) is planar.

## Patentansprüche

1. Implantat (1) für eine Osteosyntheseeinrichtung, insbesondere für die Wibelsäule, mit einem zur Verankerung im Knochen bestimmten Teil (2) und einem Körper (3) zur Befestigung an einer Stange (4), bei dem der Körper einen sich in einem hinteren Teil des Körpers öffnenden Kanal (5) aufweist, der zwei seitliche Arme (6) begrenzt und beiderseits des Körpers offen ist, um die Stange aufnehmen zu können, wobei dieses Implantat außerdem einen Gewindestopfen (10) aufweist, der in an den Innenwänden der beiden Arme (6) ausgebildete Gewinde (9) einschraubbar ist, uni die Stange (4) gegen Translation und Rotation zu sichern, dadurch **gekennzeichnet**, daß es ein Verbindungsplättchen (11) zwischen dem Stopfen (10) und der Stange (4) aufweist, das mit Mitteln (12) zur Befestigung an den Armen des Körpers versehen ist.

2. Implantat nach Anspruch 1, dadurch **gekennzeichnet**, daß die Mittel zur Befestigung des Plättchens (11) an den Armen (6) durch einen Ring (12) gebildet werden, der die genannten Arme umgibt und mit den Enden des Plättchens verbunden ist, das sich von einem Ende des Kanals (5) des Körpers (3) zum anderen erstreckt, wobei die Arme des Körpers in Durchbrüche (13) eingesteckt werden können, die zwischen dem Ring (12) und den Rändern des Plättchens (11) ausgespart sind.

3. Implantat nach Anspruch 2, dadurch **gekennzeichnet**, daß die an dem Plättchen (11) anliegende Fläche des Stopfens (10) eben ist.
